# EUROPEAN PATENT SPECIFICATION

(11) **EP 1 582 153 B1**
(45) Date of publication and mention of the grant of the patent: **22.05.2013**
(21) Application number: 04701687.8
(22) Date of filing: 13.01.2004
(51) Int. Cl.: A61B 17/12

(54) **EMBOLUS FORMING IN-VIVO INDWELLING COIL**
EMBOLUSBILDENDE IN-VIVO INNENSPIRALE
ENROULEMENT A DEMEURE IN VIVO FORMANT EMBOLE

(30) Priority: 10.01.2003 JP 2003005158
(43) Date of publication of application: 05.10.2005
(73) Proprietor: KANEKA CORPORATION, Osaka (JP); Kaneka Medix Corporation, Osaka (JP)
(72) Inventor: OGAWA, Atsushi, c/o KANEKA MEDIX CORPORATION, Ashigarakami-gun, Kanagawa 258-0113 (JP); SAKAI, Shinichi, c/o KANEKA MEDIX CORPORATION, Ashigarakami-gun, Kanagawa 258-0113 (JP)
(74) Representative: Gille Hrabal
(86) International application number: PCT/JP2004/000137
(87) International publication number: WO 2004/062510

(56) References cited:
- EP-A1- 0 792 623
- EP-A1- 0 941 701
- JP-A- 11 076 249
- JP-A- 2000 229 086

## Description

### Technical Field

The present invention relates to an embolus forming in-vivo indwelling coil, and particularly to an embolus forming in-vivo indwelling coil used for, for example, changing or interrupting a blood flow or embolizing a lesion.

### Background Art

Examples of prior art document information concerning the present invention include the following:
[Patent Document 1] Patent No. 3023076
[Patent Document 2] Patent No. 2909021
[Patent Document 3] Japanese Unexamined Patent Application Publication No. 8-187248
[Patent Document 4] PCT Japanese Translation Patent Publication No. 2002-507902
[Patent Document 5] Patent No. 2908363
[Patent Document 6] Japanese Unexamined Patent Application Publication No. 11-76249

A currently known treatment with less invasive for an aneurysm or the like is vascular embolization in which an embolus forming in-vivo indwelling device is indwelled in an aneurysm. In the vascular embolization, the embolus forming in-vivo indwelling device indwelled in the aneurysm physically interferes with a blood flow and forms a thrombus around the embolus forming in-vivo indwelling device, thereby decreasing a danger of rupture of the aneurysm. An example of the embolus forming in-vivo indwelling device which is indwelled at a predetermined position in a blood vessel, such as an aneurysm, is an embolus forming in-vivo indwelling device comprising a metal coil (referred to as an "embolus forming coil" hereinafter).

The embolus forming coil is introduced into an aneurysm through an appropriate catheter using pushing means (inductor) detachably connected to an end of the coil. Then, the embolus forming coil is separated and indwelled at the predetermined position.

However, such an embolus forming coil is required to have various characteristics: (1) In order to securely introduce and indwell the coil at the predetermined position without damaging a blood vessel, the coil has high flexibility for permitting the coil to be deformed along the shape of a blood vessel. (2) The coil has the function to prevent or suppress unrestricted stretch so as to permit a secured re-indwelling operation for recovering the coil and correcting the indwelling position after the coil has been pushed out from the catheter and placed in a blood vessel. Namely, for example, when the embolus forming coil gets caught on the tip end of the catheter and extended thereby, the embolus forming coil cannot be easily recovered or the indwelling position may be damaged. Therefore, the embolus forming coil must be formed so as not to cause this problem.

EP 0 971 701 A discloses an embolic coil to be placed at a preselected location within a vessel comprising a helically wound coil in which various combinations of adjacent turns are spot welded together to create a stretch resistant coil of a preselected flexebility.

### Disclosure of Invention

The present invention has been achieved on the basis of the above-described situation, and an object of the present invention is to provide an embolus forming in-vivo indwelling coil which has so high flexibility that the coil can be securely introduced and indwelled at a predetermined position in the body, and which permits a secured re-indwelling operation including recovery of the in-vivo indwelling device, for example, for recovering the disposed device and correcting the position, and thus has high safety and high operationality.

An embolus forming in-vivo indwelling coil of the present invention comprises a coil main body having flexibility and an stretch suppressing member which is provided on one or both of the inner and outer peripheries of the coil main body and which is made of a water-swellable polymer material for suppressing stretch of the coil main body by swelling with absorbed water.

In the embolus forming in-vivo indwelling coil of the present invention, the water-swellable polymer material constituting the stretch suppressing member preferably comprises a polyvinyl alcohol polymer.

In addition, preferably, the wire constituting the coil main body has a diameter of 10 to 120 µm, and the coil main body has a coil diameter of 100 to 500 µm, a coil length of 2 to 500 mm, and a number of turns of 1 to 100 per unit length (1 mm).

Specifically, the rod-like or cylindrical stretch suppressing member is provided in the coil main body so as to pass through the coil main body and extend in the coil axial direction of the coil main body. In this case, the diameter of the stretch suppressing member is preferably smaller than the inner diameter of the coil main body by about 1 to 50% in a dry state.

Alternatively, the cylindrical or tubular stretch suppressing member may be provided to cover the entire region of the outer periphery of the coil main body in the coil axial direction. In this case, in a dry state, the thickness of the stretch suppressing member is preferably 0.01 to 0.10 mm, and the clearance between the outer periphery of the coil main body and the inner periphery of the stretch suppressing member is preferably 0 to 100 µm.

The embolus forming in-vivo indwelling coil of the present invention may comprise a rod-like or cylindrical stretch suppressing member provided so as to extend in the coil axial direction of a coil main body and pass through the coil main body, and a cylindrical or tubular stretch suppressing member provided to cover the entire region of the outer periphery of the coil main body in the coil axial direction.

In an indwelling operation, the embolus forming in-vivo indwelling coil of the present invention is used in a swollen state in which the stretch suppressing member is previously swollen. As a result of swelling, the stretch suppressing member enters the coil pitches (wire spaces) in the inner periphery or the outer periphery of the coil main body to create a state in which the adjacent wire turns are substantially connected to each other.

However, in the swollen state, the stretch suppressing member has deformability, and thus, basically, the flexibility of the coil main body is not significantly inhibited by the stretch suppressing member. Therefore, the embolus forming in-vivo indwelling coil can be formed with high flexibility, and high operationality can be obtained in the indwelling operation. As a result, the embolus forming in-vivo indwelling coil can be securely introduced and indwelled at a predetermined position through an appropriate catheter.

Furthermore, for example, even in a re-indwelling operation for recovering the disposed coil and correcting the indwelling position, stretch of the coil main body in the coil axial direction is restricted because the wire turns in the coil main body are substantially connected to each other by the stretch suppressing member. In this state, the embolus forming in-vivo indwelling coil is pulled back into the catheter, and thus the re-indwelling operation including recovery of the in-vivo indwelling coil can be securely performed. Therefore, the embolus forming in-vivo indwelling coil can be formed with high safety.

### Brief Description of the Drawings

Fig. 1 is a sectional view schematically illustrating the configuration of an example of an embolus forming in-vivo indwelling coil of the present invention.
Fig. 2 is a sectional view illustrating a state in which an stretch suppressing member of the embolus forming in-vivo indwelling coil shown in Fig. 1 is swollen.
Fig. 3 is a sectional view schematically illustrating the configuration of another example of the embolus forming in-vivo indwelling coil of the present invention.
Fig. 4 is a sectional view illustrating a state in which an stretch suppressing member of the embolus forming in-vivo indwelling coil shown in Fig. 3 is swollen.
Fig. 5 is an enlarged sectional view illustrating the configuration of still another example of the embolus forming in-vivo indwelling coil of the present invention.
Fig. 6 is an enlarged sectional view illustrating the configuration of a further example of the embolus forming in-vivo indwelling coil of the present invention.
Fig. 7 is a drawing illustrating a method for measuring the flexibility of an in-vivo indwelling device according to the present invention.

### Best Mode for Carrying Out the Invention

The present invention will be described in detail below with reference to the drawings.

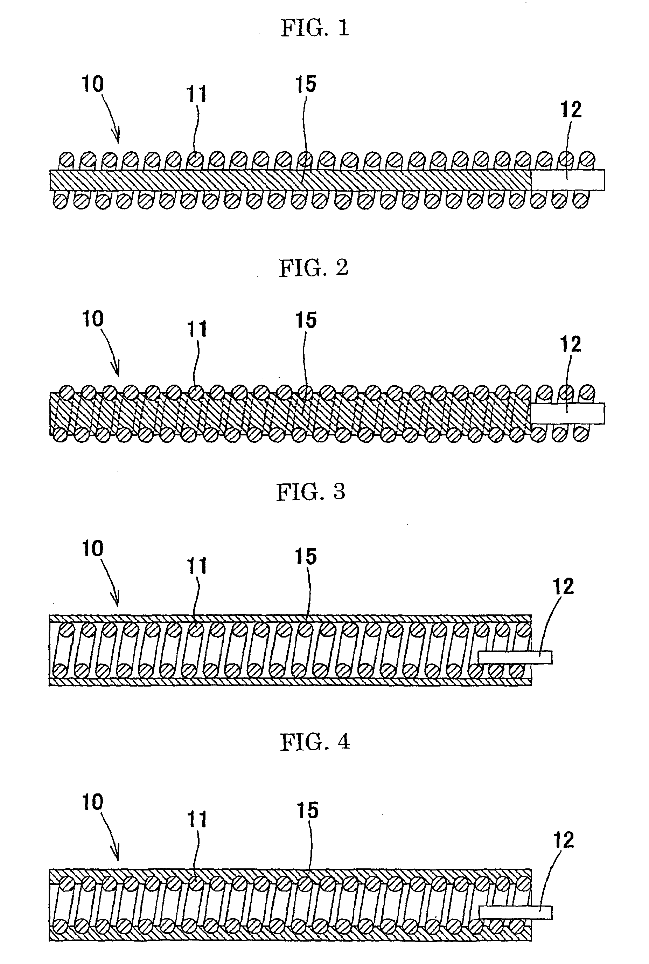

Fig. 1 is a sectional view schematically illustrating the configuration of an example of an embolus forming in-vivo indwelling coil of the present invention.

The embolus forming in-vivo indwelling coil (simply referred to as an "in-vivo indwelling coil" hereinafter) 10 comprises a coil main body 11 having flexibility. Also, for example, a rod-shaped coil separating member 12 for holding the coil main body 11 is provided at the proximal end (the right-side end in Fig. 1) of the coil main body 11 so as to be partially fixed on the inner periphery of the coil main body 11 at the proximal end to project outward (rightward in Fig. 1) from the proximal end of the coil main body 11 in the coil axial direction.

As a wire for the coil main body 11, any one of various materials, for example, a metal wire, a resin wire, and the like, can be used as long as the wire does not adversely affect a human body (having biocompatibility) when being indwelled in the human body for a long time.

Examples of the metal wire for forming the coil main body 11 include tungsten, gold, platinum, and stainless steel, and alloys thereof.

Examples of the resin wire include polyester resins such as polyethylene terephthalate (PET), polyolefin resins such as polypropylene (PP), and polyamide resins such as nylon.

As described above, the coil main body 11 constituting the in-vivo indwelling coil 10 has flexibility and preferably has the structure below depending upon the material of the wire used for forming the coil main body 11.

For example, the diameter (element wire diameter) of the wire constituting the coil main body 11 is 10 to 120 µm, and the coil main body 11 has a coil diameter of 100 to 500 µm, a coil length of 2 to 500 mm, and a number of turns of 1 to 100 per unit length (1 mm).

The in-vivo indwelling coil 10 further comprises an stretch suppressing member 15 of, for example, a rod shape, which is made of a water-swellable polymer material and functions to suppress stretch of the coil main body 11 by swelling with absorbed water, and which is provided to extend in the coil axial direction of the coil main body 11 and to pass through the coil main body 11 (through a lumen).

As the polymer material constituting the stretch suppressing member 15, any material which has proper water-swellability and no adverse effect on human bodies can be used. Specific examples of the polymer material include polyvinyl alcohol polymers; natural polysaccharides such as carrageenan, agar, alginic acid, starch, pectin, galactomannan, xanthane, hyaluronic acid, chitin, and chitosan; natural proteins such as collagen, actin, myosin, tublin, casein, and fibrin; chemically-modified natural polymers such as carboxymethylated cellulose, carboxymethylated starch, acrylic acid-grafted starch, acrylonitrile-grafted cellulose hydrolyzates; and synthetic polymers such as polyacrylic acid, polyvinyl alcohol, polyethyleneimine, polyhydroxyethyl methacrylate, polyvinylpyrrolidone, and polyethylene oxide. Among these materials, polyvinyl alcohol polymers are preferred because a crystalline substance can be easily oriented, and excellent physical strength can be achieved by orienting a crystalline substance.

The diameter of the stretch suppressing member 15 depends on the water-swellability of the material of the stretch suppressing member 15, but the diameter may be determined so that in a dry state, it can be inserted into the coil main body (lumen). For example, the diameter is preferably smaller than the coil diameter of the coil main body 11 by about 1 to 50%.

From the viewpoint that a desired stretch preventing effect is securely expressed in a swollen state, the stretch suppressing member 15 is preferably disposed to extend over the entire region of the coil main body 11 in the coil axial direction. In other words, the stretch suppressing member 15 preferably has a length which is the same as or larger than the coil length of the coil main body 11.

As shown in Fig. 3, in the in-vivo indwelling coil 10 of the present invention, for example, the cylindrical or tubular stretch suppressing member 15 may be provided to cover the entire region of the outer periphery of the coil main body 11 in the coil axial direction.

The thickness of the stretch suppressing member 15, which constitutes the in-vivo indwelling coil 10, can be properly determined to express the intended stretch preventing effect in a swollen state. Specifically, in a dry state, the diameter is preferably 0.01 to 0.10 mm, and the clearance between the outer periphery of the coil main body 11 and the inner periphery of the stretch suppressing member 15 is preferably 0 to 100 µm.

The above-described in-vivo indwelling coil 10 can be also used in a state in which the linear primary form is maintained. However, in view of improvement in the embolus forming ability at a predetermined position, the coil 10 is preferably used, for example, in a state in which the linear coil main body 11 is helically coiled to form a secondary coil or in a state in which the coil main body 11 is formed in a S- or J-like three-dimensional shape, or another secondary shape.

The method for using the in-vivo indwelling coil 10 will be described below with reference to an example in which the coil 10 is applied to treatment of an aneurysm.

First, the stretch suppressing member 15 constituting the in-vivo indwelling coil 10 is swollen by, for example, immersing in appropriate swelling water.

The swelling water is not particularly limited as long as it is not harmful to organisms. For example, physiological saline, purified water, extra-pure water, ion-exchanged water, an aqueous solution of a compound such as dimethylsulfoxide, blood, or the like can be used.

In swelling the stretch suppressing member 15, the time of immersion of the in-vivo indwelling coil 10 in the swelling water is preferably, for example, 5 to 180 seconds depending on the constitutive material and configuration of the stretch suppressing member 15, and the like.

When the stretch suppressing member 15 of the in-vivo indwelling coil 10 is swollen, the stretch suppressing member 15 enters the coil pitches (wire spaces) in the inner periphery of the coil main body 11 as shown in Fig. 2, or the stretch suppressing member 15 enters the coil pitches in the outer periphery of the coil main body 11 as shown in Fig. 4. Namely, the adjacent turns of the wire, which constitutes the coil main body 11, are substantially connected to each other. In this state, the indwelling coil 10 is formed in a secondary shape, for example, a secondary coil, and then detachably mounted on the tip of a guide wire.

Examples of a method for separating the in-vivo indwelling coil 10 from the guide wire include a mechanical separating method (1) in which the coil separating member 12 of the in-vivo indwelling coil 10 is mechanically engaged with the guide wire, and an electric separating method (2) in which for example, a monopolar high-frequency current is supplied to melt and disconnect the coil separating member 12 of the in-vivo indwelling coil 10 by heating with the high-frequency current, thereby separating the in-vivo indwelling coil 10 from the guide wire.

Then, a proper catheter is endermically inserted into a blood vessel using a puncture needle until the end of the catheter reaches the inlet of an aneurysm. Thereafter, the in-vivo indwelling coil 10 is linearly stretched and returned to the primary form (shown in Fig. 2 or 4). In this state, the guide wire is inserted into the catheter and moved forward to push out the in-vivo indwelling coil 10 from the end of the catheter and dispose the in-vivo indwelling coil 10 in the aneurysm.

When the in-vivo indwelling coil 10 is push out from the catheter, it is restored to the secondary coil, which is a secondary form, in which the in-vivo indwelling coil 10 is three-dimensionally tangled. In this state, the complete insertion of the in-vivo indwelling coil 10 into the aneurysm is confirmed by radioscopy. Then, the in-vivo indwelling coil 10 is separated from the end of the guide wire and indwelled in the aneurysm.

If required, the above-described indwelling operation is repeated using a plurality of the in-vivo indwelling coils 10 to fill the aneurysm with a plurality of in-vivo indwelling coils 10 and form a thrombus, thereby inhibiting a blood inflow to the aneurysm. As a result, rupture of the aneurysm can be securely prevented.

In the indwelling operation, the in-vivo indwelling coil 10 of the present invention is used after the stretch suppressing member 15 is previously swollen. When the stretch suppressing member 15 is swollen, therefore, the stretch suppressing member 15 enters the wire spaces in the inner periphery or the outer periphery of the coil main body 11 to create a state in which the adjacent wire turns are substantially connected to each other.

Specifically, in the in-vivo indwelling coil 10 having the structure shown in Fig. 1, the stretch suppressing member 15 is swollen to fill in the coil main body 11 (lumen) and enters the coil pitches in the inner periphery of the coil main body 11, as shown in Fig. 2. In the in-vivo indwelling coil 10 having the structure shown in Fig. 3, the stretch suppressing member 15 is swollen to enter the coil pitches from the outer periphery of the coil main body 11, thereby causing a state in which the adjacent wire turns are substantially connected to each other. As a result, the coil main body 11 is supported by the stretch suppressing member 15.

Therefore, the stretch suppressing member 15 is formed with deformability in a swollen state, and thus, basically, the flexibility of the coil main body 11 is not significantly inhibited by the stretch suppressing member 15. Therefore, the in-vivo indwelling coil 10 can be formed with high flexibility, and high operationality of the indwelling operation can be achieved. Consequently, the in-vivo indwelling coil 10 can be securely introduced and indwelled at the predetermined position through the appropriate catheter.

Furthermore, when a re-indwelling operation is required for recovering the disposed coil and correcting the indwelling position, the in-vivo indwelling coil 10 of the present invention is returned into the catheter in the state in which stretch of the coil main body in the coil axial direction is restricted because the adjacent turns of the wire constituting the coil main body 11 are substantially connected to each other by the stretch suppressing member 15. Therefore, the re-indwelling operation including recovery of the in-vivo indwelling coil 10 can be securely performed. As a result, the in-vivo indwelling coil 10 can be formed with high safety.

In the in-vivo indwelling coil having the structure shown in Fig. 3, the stretch suppressing member 15 is provided over the entire region of the outer periphery of the coil main body 11, and the stretch suppressing member 15 is made of a flexible material having deformability in a swollen state. Therefore, the lubricating effect is exhibited in moving the in-vivo indwelling coil 10 inserted in the catheter to the predetermined position, thereby achieving high operationality. Also, since the coil main body 11 is not exposed, damage to the applied position can be securely prevented to achieve the function to protect the applied position.

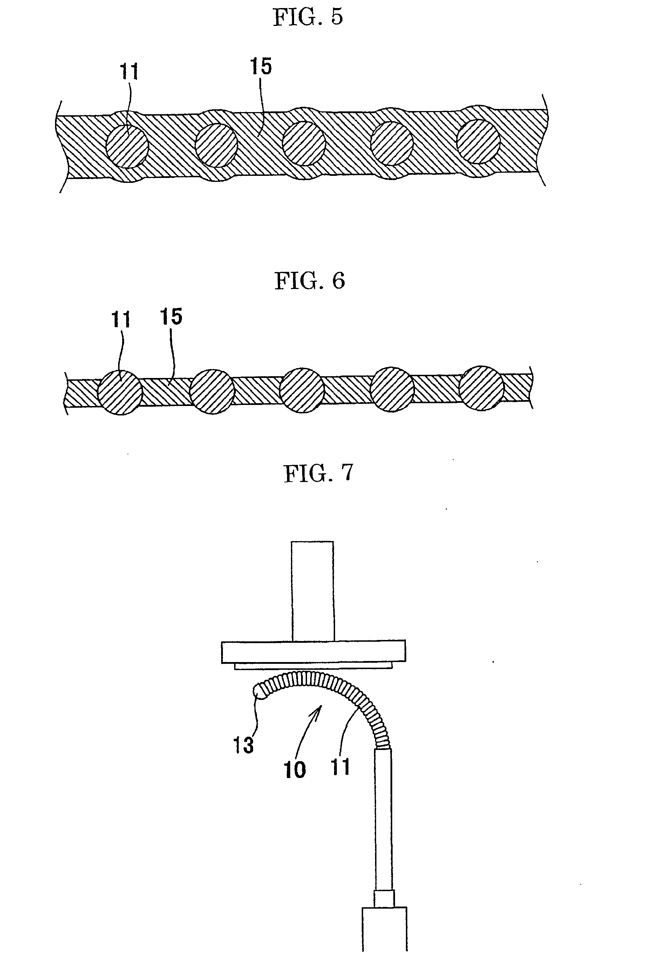

The preferred embodiments of the present invention are described above, but the present invention is not limited to these embodiments, and the modifications below can be made.
(1) An stretch suppressing member may be disposed so as to realize a state in which in a swollen state, the stretch suppressing member enters the spaces of a wire constituting a coil main body. For example, as shown in Fig. 5, the stretch suppressing member may be formed into a film so as to cover the entire surface of the wire in a dry state and connect the adjacent wire turns. Alternatively, as shown in Fig. 6, the stretch suppressing member may be formed into a film so as to fill in wire spaces in a dry state. In these structures, the stretch suppressing member is put into a flexible state with deformability when being swollen, and thus stretch of the coil main body can be securely restricted by the stretch suppressing member without significantly inhibiting the flexibility of the coil main body.
   In the in-vivo indwelling coil shown in Fig. 1, the stretch suppressing member may be formed, for example, in a cylindrical shape. In this case, the specific structural conditions of the stretch suppressing member, such as the thickness and the outer diameter dimension thereof, and the like, may be properly determined so that the intended stretch suppressing function can be exhibited.
(2) Although not shown in a drawing, the in-vivo indwelling coil of the present invention may comprise an internal stretch suppressing member inserted into a coil main body and an external stretch suppressing member provided to cover the entire surface of the outer periphery of the boil main body. In this case, the internal and external stretch suppressing members are swollen to enter the coil pitches in the coil main body from both the inner side and the outer side, thereby forming a state in which the adjacent wire turns can be substantially connected to each other. Therefore, a higher stretch suppressing effect on the coil main body can be obtained. When the internal stretch suppressing member is formed in, for example, a cylindrical shape, the intended stretch suppressing effect can be securely exhibited without significantly increasing the time required for swelling.
(3) A coil main body may comprise a fiber material, for example, polyester or the like, which is woven into a loop and provided on the coil main body, or a fiber material woven into a cylindrical form to cover the outer surface of the coil main body. In this case, the in-vivo indwelling coil can be formed with a higher embolus forming ability.

### <Experimental Example>

The experimental examples conducted for confirming the functional effect of the in-vivo indwelling coil of the present invention will be described below.

### [Production Example 1]

A rod-shaped stretch suppressing member comprising polyvinyl alcohol and having a diameter of 120 µm and a length of 50 mm was inserted into a metal coil (coil diameter: 250 µm, coil length: 50 mm, number of turns per 1 mm: 20) which was formed by coiling a tungsten wire having an element wire diameter of 50 µm. As a result, an in-vivo indwelling coil having the structure shown in Fig. 1 was produced. This is referred to as "indwelling coil 1".

### [Production Example 2]

A metal coil having the same structure as in Production Example 1 was formed, and a cylindrical stretch suppressing member comprising polyvinyl alcohol and having a diameter of 350 µm, a thickness of 50 µm, and a length of 50 mm was disposed to cover the entire region of the outer periphery of the metal coil to produce an in-vivo indwelling coil having the structure shown in Fig. 3. This is referred to as "indwelling coil 2".

### [Production Example 3]

A metal coil having the same structure as in Production Example 1 was formed, and a rod-shaped stretch suppressing member comprising polyvinyl alcohol and having a diameter of 120 µm and a length of 50 mm was inserted into the metal coil. Furthermore, a cylindrical stretch suppressing member comprising polyvinyl alcohol and having a diameter of 350 µm, a thickness of 50 µm, and a length of 50 mm was disposed to cover the entire region of the outer periphery of the metal coil was disposed to cover the entire region of the outer periphery of the metal coil to produce an in-vivo indwelling coil. This is referred to as "indwelling coil 3".

Each of the indwelling coils 1 to 3 produced as described above was subjected to swelling of the stretch suppressing member under the conditions shown in Table 1, and then evaluated with respect to (1) flexibility of the whole indwelling coil and (2) the stretch suppressing function of the stretch suppressing member on the coil main body in a swollen state. The results are shown in Table 1.

### [Evaluation]

(1) The flexibility of the whole indwelling coil was determined as follows:
   As shown in Fig. 7, the in-vivo indwelling coil 10 was fixed with a tube so that the primary coil axis of the coil main body 11 was extended in, for example, the vertical direction (the longitudinal direction in Fig. 7). In a state in which any portion in the range from half pitch to one pitch of a secondary coil was exposed, a load was slowly applied to the in-vivo indwelling coil 10 from above in the coil axial direction, and the stress produced when the portion in the range from half pitch to one pitch was bent at the end of the coil main body 11 was measured as compressive elasticity modulus. In Fig. 7, reference numeral 13 denotes the rounded head having a substantially hemispherical shape and provided at the distal end of the in-vivo indwelling coil 10.
(2) Each of the indwelling coils subjected to swelling was measured with respect to the coil length of the coil main body when a load of 80 mN/mm² was applied outward in the coil axial direction of the coil main body, and an elongation of the coil main body was calculated.

**Table 1**

| | Swelling conditions | | Flexibility of whole indwelling coil (mN/mm²) | Elongation of coil main body (mm) |
|---|---|---|---|---|
| | Swelling water | Rate of volume increase (%) | | |
| Indwelling coil 1 | Purified water | 185 | 8 | 3 |
| Indwelling coil 2 | Physiological saline | 115 | 8 | 3 |
| Indwelling coil 3 | Purified water | 123 | 10 | 2 |

The results confirm that in the indwelling coils 1 to 3 of the present invention, the elongation of the coil main body in the coil axial direction is restricted to 3 mm or less, and the intended stretch suppressing effect is securely exhibited, and that the in-vivo indwelling coils have high flexibility. Therefore, it can be expected that high operationality and high safety are obtained in an indwelling operation, and the indwelling operation can be reliably performed.

### Industrial Applicability

In an indwelling operation, an embolus forming in-vivo indwelling coil of the present invention is used in a state in which an stretch suppressing member is previously swollen. As a result of swelling, the stretch suppressing member enters the coil pitches (wire spaces) in the inner periphery or the outer periphery of the coil main body to create a state in which the adjacent wire turns are substantially connected to each other.

However, in the swollen state, the stretch suppressing member has deformability, and thus, basically, the flexibility of the coil main body is not significantly inhibited by the stretch suppressing member. Therefore, the embolus forming in-vivo indwelling coil can be formed with high flexibility, and high operationality can be obtained in the indwelling operation. As a result, the embolus forming in-vivo indwelling coil can be securely introduced and indwelled at a predetermined position through an appropriate catheter.

Furthermore, for example, even in a re-indwelling operation for recovering the disposed coil and correcting the indwelling position, stretch of the coil main body in the coil axial direction is restricted because the wire turns in the coil main body are substantially connected to each other by the stretch suppressing member. In this state, the embolus forming in-vivo indwelling coil is pulled back into the catheter, and thus the re-indwelling operation including recovery of the in-vivo indwelling coil can be securely performed. Therefore, the embolus forming in-vivo indwelling coil can be formed with high safety.

## Claims

1. An embolus forming in-vivo indwelling coil (10) comprising a coil main body (11) constituted by a wire, having flexibility and a member (15) disposed to extend in the coil axial direction of the coil main body (11), **characterized in that**
the member (15) made of a water-swellable polymer material and provided on one or both of the inner and outer peripheries of the coil main body (11) becomes a stretch suppressing member (15) for suppressing stretch of the coil main body (11), wherein the member (15) enters the wire spaces and create a state in which the adjacent wire turns are substantially connected to each other by swelling with absorbed water.

2. The embolus forming in-vivo indwelling coil (10) according to claim 1, wherein the water-swellable polymer material constituting the stretch suppressing member (15) comprises a polyvinyl alcohol polymer.

3. The embolus forming in-vivo indwelling coil (10) according to claim 1 or 2, wherein the wire constituting the coil main body (11) has a diameter of 10 to 120 µm, and the coil main body (11) has a coil diameter of 100 to 500 µm, a coil length of 2 to 500 mm, and a number of turns of 1 to 100 per unit length (1 mm).

4. The embolus forming in-vivo indwelling coil (10) according to any one of claims 1 to 3, wherein the stretch suppressing member (15) has a rod-like shape or cylindrical shape and is provided in the coil main body (11) so as to pass through the coil main body (11) and extend in the coil axial direction of the coil main body (11).

5. The embolus forming in-vivo indwelling coil (10) according to claim 4, wherein the diameter of the stretch suppressing member (15) is smaller than the inner diameter of the coil main body (11) by about 1 to 50% in a dry state.

6. The embolus forming in-vivo indwelling coil (10) according to any one of claims 1 to 3, wherein the stretch suppressing member (15) has a cylindrical or tubular shape and is provided to cover the entire region of the outer periphery of the coil main body (11) in the coil axial direction.

7. The embolus forming in-vivo indwelling coil (10) according to claim 6, wherein, in a dry state, the thickness of the stretch suppressing member (15) is 0.01 to 0.10 mm, and the clearance between the outer periphery of the coil main body (11) and the inner periphery of the stretch suppressing member (15) is 0 to 100 µm.

8. The embolus forming in-vivo indwelling coil (10) according to any one of claims 1 to 3, wherein a stretch suppressing member (15) has a rod-like or cylindrical shape and is provided so as to extend in the coil axial direction of a coil main body (11) and pass through the coil main body (11), and another stretch suppressing member (15) has a cylindrical or tubular shape and is provided to cover the entire region of the outer periphery of the coil main body (11) in the coil axial direction.

## Patentansprüche

1. Eine Embolus-bildende in-vivo Innenspirale (10), umfassend einen Spiralenhauptkörper (11) gebildet aus einem flexiblen Draht und einem Element (15), welches angeordnet ist, um sich in der Spirale in axialer Richtung des Spiralenhauptkörpers (11) zu erstrecken, **dadurch gekennzeichnet, dass**
das Element (15), welches aus einem wasserquellbaren Polymermaterial hergestellt wird und an einer oder beiden der inneren und äußeren Umfänge des Spiralenhauptkörpers angebracht ist, ein die Dehnung unterdrückendes Element (15) wird zur Unterdrückung der Dehnung des Spiralenhauptkörper (11) wobei das Element (15) in die Draht-Zwischenräume eindringt und einen Zustand erzeugt, in dem die benachbarten Drahtwindungen durch Quellung mit absorbiertem Wasser im Wesentlichen miteinander verknüpft sind.

2. Die Embolus-bildende in-vivo Innenspirale (10) nach Anspruch 1, worin das wasserquellbare Polymermaterial, aus dem das die Dehnung unterdrückenden Element gebildet wird, ein Polyvinylalkoholpolymer umfasst.

3. Die Embolus-bildende in-vivo Innenspirale (10) nach Anspruch 1 oder 2. worin der Draht, aus welchem der Spiralenhauptkörper (11) besteht, einen Durchmesser von 10 bis 120 µm hat und der Spiralenhauptkörper (11) einen Spiralendurchmesser von 100 bis 500 µm, eine Spiralenlänge von 2 bis 500 mm und eine Windungsanzahl von 1 bis 100 pro Längeneinheit (1 mm) aufweist.

4. Die Embolus-bildende in-vivo Innenspirale (10) nach einem der Ansprüche 1 bis 3, worin das die Dehnung unterdrückende Element (15) eine stabförmige oder zylindrische Form aufweist und in dem Spiralenhauptkörper (11) so angebracht ist, dass es den Spiralenhauptkörper (11) durchdringt und sich In der Spirale in axialen Richtung des Spiralenhauptkörpers (11) erstreckt.

5. Die Embolus-bildende in-vivo Innenspirale (10) nach Anspruch 4. worin der Durchmesser des die Dehnung unterdrückenden Elements (15) im trockenen Zustand um etwa 1 bis 50% kleiner ist als der innere Durchmesser des Spiralenhauptkörpers (11).

6. Die Embolus-bildende in-vivo Innenspirale (10) nach einem der Ansprüche 1 bis 3, worin das die Dehnung unterdrückende Element (15) eine zylindrische oder röhrenförmige Gestalt hat und so angebracht ist, dass es den gesamten Bereich des äußeren Umfangs des Spiralenhauptkörpers (11) in axialer Richtung der Spirale abdeckt.

7. Die Embolus-bildende in-vivo Innenspirale (10) nach Anspruch 6, worin die Dicke des die Dehnung unterdrückenden Elements (15) im trockenen Zustand 0,01 bis 0,10 mm ist und der Abstand zwischen dem äußeren Umfang des Spiralenhauptkörpers (11) und dem Innenumfang des die Dehnung unterdrückenden Elements (15) 0 bis 100 µm beträgt.

8. Die Embolus-bildende in-vivo Innenspirale (10) nach einem der Ansprüche 1 bis 3, worin ein die Dehnung unterdrückendes Element (15) eine stabförmige oder zylindrische Form aufweist und so angebracht ist, dass es sich in axialer Richtung der Spirale des Spiralenhauptkörpers (11) erstreckt und den Spiralenhauptkörper (11) durchdringt und ein anderes die Dehnung unterdrückendes Element (15) eine zylindrische oder röhrenförmige Gestalt aufweist und so angebracht ist, dass es den gesamten Bereich des äußeren Umfangs des Spiralenhauptkörpers (11) in axialer Richtung abdeckt.

## Revendications

1. Enroulement à demeure in vivo formant un embole (10) comprenant un corps principal d'enroulement (11) constitué par un fil flexible et un élément (15) disposé afin qu'il étend dans la direction axiale d'enroulement du corps principal d'enroulement (11) **caractérisé en ce que**
l'élément (15) est fait d'un matériau d'un polymère gonflable à l'eau et disposé sur une ou les deux périphéries à l'intérieur et à l'extérieur du corps principal d'enroulement (11) devient un élément de suppression d'expansion (15) pour supprimer l'expansion du corps principal d'enroulement (11), dans lequel l'élément (15) entre l'espace du fil et crée un état, dans lequel les spires du fil adjacents sont connectés substantiellement entre eux par la gonflage avec de l'eau absorbé.

2. Enroulement à demeure in vivo formant un embole (10) selon la revendication 1, dans lequel le matériau d'un polymère gonflable à l'eau constituant l'élément de suppression d'expansion (15) comprend un polymère d'un alcool polyvinylique.

3. Enroulement à demeure in vivo formant un embole (10) selon l'une des revendications 1 ou 2, dans lequel le fil constituant le corps principal d'enroulement (11) a un diamètre de 10 à 120 µm et le corps principal d'enroulement (11) a un diamètre d'enroulement de 100 à 500 µm, une longueur d'enroulement de 2 à 500 mm et un nombre des spires de 1 à 100 par unité de longueur (1 mm).

4. Enroulement à demeure in vivo formant un embole (10) selon l'une des revendications 1 à 3, dans lequel l'élément de suppression d'expansion (15) a une forme de barre ou une forme cylindrique et est disposé dans le corps principal d'enroulement (11) pour traverser le corps principal d'enroulement (11) et étendre dans la direction axiale d'enroulement du corps principal d'enroulement (11).

5. Enroulement à demeure in vivo formant un embole (10) selon la revendication 4, dans lequel le diamètre de l'élément de suppression d'expansion (15) est inférieur au diamètre intérieur du corps principal d'enroulement (11) d'environ 1 à 50 % à l'état sec.

6. Enroulement à demeure in vivo formant un embole (10) selon l'une des revendications 1 à 3, dans lequel l'élément de suppression d'expansion (15) a une forme cylindrique ou tubulaire et est pourvu pour couvrir toute la région de la périphérie à l'extérieur du corps principal d'enroulement (11) dans la direction axiale d'enroulement.

7. Enroulement à demeure in vivo formant un embole (10) selon la revendication 6, dans lequel, à l'état sec, l'épaisseur de l'élément de suppression d'expansion (15) est 0,01 à 0,10 mm et le dégagement entre la périphérie à l'extérieur du corps principal d'enroulement (11) et la périphérie à l'intérieur de l'élément de suppression d'expansion (15) est 0 à 100 µm.

8. Enroulement à demeure in vivo formant un embole (10) selon l'une des revendications 1 à 3, dans lequel l'élément de suppression d'expansion (15) a une forme de barre ou une forme cylindrique et est disposé afin qu'il étend dans la direction axiale d'enroulement du corps principal d'enroulement (11) et traverse le corps principal d'enroulement (11) et un autre l'élément de suppression d'extension (15) a une forme cylindrique ou tubulaire et est pourvu pour couvrir toute la région de la périphérie à l'extérieur du corps principal d'enroulement (11) dans la direction axiale d'enroulement.
